Europäisches Patentamt

(19) European Patent Office

Office européen des brevets

(11) Publication number: **0 008 121**
**B1**

(12) **EUROPEAN PATENT SPECIFICATION**

(45) Date of publication of patent specification: **23.02.83**

(21) Application number: **79102884.8**

(22) Date of filing: **09.08.79**

(51) Int. Cl.³: **A 01 N 37/04, C 12 N 7/06**

(54) **Use of glutaric acid as a virucidal agent and a composition containing it.**

(30) Priority: **14.08.78 US 933602**

(43) Date of publication of application:
**20.02.80 Bulletin 80/4**

(45) Publication of the grant of the patent:
**23.02.83 Bulletin 83/8**

(84) Designated Contracting States:
**BE CH DE FR GB IT NL SE**

(56) References cited:
**FR - A - 2 227 861**

**CHEMICAL ABSTRACTS, vol. 68, no. 23, June 3, 1968; page 9903, abstract 102650h, Columbus, Ohio, USA**
**A. GERMAN: "The use of Stephylococcus Aureus to study antiviral substances".**

(73) Proprietor: **STERLING DRUG INC.**
**90 Park Avenue**
**New York New York (US)**

(72) Inventor: **Guy, Dominic Diana**
**Box 192, Garfield Road**
**East Nassau, New York (US)**

(74) Representative: **Baillie, Iain Cameron et al,**
**c/o Ladas & Parry Isartorplatz 5**
**D-8000 München 2 (DE)**

(56) References cited:
**CHEMICAL ABSTRACTS, vol. 71, no. 11, September 15, 1969, page 203, abstract 47931k. Columbus, Ohio, USA**
**F. SABEL "Glutaraldehyde inactivation of virus in tissue".**

Courier Press, Leamington Spa, England

**0 008 121**

Use of glutaric acid as a virucidal agent and a
composition containing it

This invention relates to a process and compositions for neutralizing or destroying a virus.

It has been shown (Hendley, Wenzel and Gwaltney, New England Journal of Medicine, vol. 288, pp. 1361—1364, 1973) that colds caused by rhinoviruses can be transmitted from person to person or from person to object to person by way of the hands. It is believed that self-infection takes place by transfer of virus from the fingers to the nasal mucosa or the conjunctiva. Similar transmittal of other types of virus is also believed possible. The resulting need for a topical virucidal substance and method of use and formulations thereof, especially for the hands, is satisfied by the present invention.

Substantivity would also be desirable in a new, topical virucidal substance, whereby the topically applied substance would have virucidal effect against a subsequently applied virus. The present invention also satsifies this need.

Glutaric acid is a well-known organic compound having the structural formula

$$HOOCCH_2CH_2CH_2COOH$$

which is not known for its pharmaceutical uses, microbiocidal or other. In fact, the literature (Gershon and Shanks, Canadian Journal of Microbiology, vol. 22, no. 8, pp. 1198—1201, 1976; Chemical Abstracts, vol. 85, 117344m, 1976) describes the antifungal testing of the alkanedioic acids of 2—4, 14 and 16 carbon atoms and the dimethyl diesters thereof and states that "[t]he dicarboxylic acids possessed very poor to no antifungal activity against all 6 fungi" (Aspergillis niger, Trichoderma veride, Myrothecium verrucaria, Candida albicans, Trichophyton mentagrophytes, Mucor mucedo). Its property as a virucidal substance has been heretofore unknown. The present invention reflects the discovery of this property.

One aspect of the present invention deals with a process of neutralizing or destroying a susceptible virus which comprises contacting the virus with a virucidally effective amount of glutaric acid.

A further aspect the invention is a composition for neutralizing or destroying a susceptible virus which consists essentially of a virucidally effective concentration of glutaric acid and a vehicle.

Virucidal is defined (Dorland's Illustrated Medical Dictionary, Twenty-fifth Edition, 1974) as "capable of neutralizing or destroying a virus". In the present invention a susceptible virus is considered to mean one which is neutralized or destroyed by glutaric acid. The susceptible viruses are readily identified in tests such as those described below, wherein the amount or concentration of glutaric acid is considered virucidal if the virus titer is reduced by 99.9% or more.

Tests of Glutaric Acid for Virucidal Activity Against Rhinoviruses
*Viruses*

Rhinoviruses types 2, 5, 13, 14, 22, 41 and 50 were obtained from the National Institute of Allergy and Infectious Diseases, were propagated in a continuous line of HeLa (Ohio) cells, and contained the following titers of virus.

| Rhinovirus Type | $Log_{10}TCID_{50}$ per 0.2 ml. |
|:---:|:---:|
| 2 | 6.5 |
| 5 | 5.25 |
| 13 | 6.25 |
| 14 | 6.25 |
| 22 | 5.5 |
| 41 | 6.0 |
| 50 | 6.25 |

Rhinovirus Type 39 was obtained from the University of Virginia School of Medicine, was also propagated in HeLa (Ohio) cells, and contained $log_{10}$ 6.0 $TCID_{50}$ per 0.2 ml. of virus.

*Virus Assay*

Virus titrations were conducted in monolayers of HeLa (Ohio) cells. Two-tenths ml. of virus dilution was inoculated into quadruplicate tubes and allowed to incubate for 1 hr. at 33°C. Eight-tenths ml. of maintenance medium (M-199) supplemented with 5% fetal calf serum was added and the cultures were incubated at 33°C. Each tube was examined for viral cytopathic effect at 96 hours and, for rhinovirus type 2, also at 72 hrs.

*Glutaric Acid*

The glutaric acid used was a commercial grade of glutaric acid.

Suspension Test for Virucidal Activity Against Rhinovirus Type 2

Glutaric acid was dissolved in maintenance medium (M-199) at concentrations of 0.5% and 1%. The pH was adjusted to 6.0—7.0 with either sodium hydroxide or hydrochloric acid as required. Two-tenths ml. of undiluted virus was mixed with 1.8 ml. of the 0.5% or 1% glutaric acid solution. The mixtures were allowed to stand for ten minutes at room temperature, then diluted and assayed. The virus titers were reduced by >99.99% at both concentrations of glutaric acid.

Rabbit Skin Test for Residual Virucidal Activity Against Rhinovirus Type 2

The dorsal skin of 2—3 kg. albino rabbits of either sex was used. Hair was removed by clipping and the skin was cleansed with warm water. Two circular areas, each 3 cm. in diameter, were drawn on either side of the midline of the back with indelible ink. The entire dorsal area was then scrubbed with 70% ethyl alcohol and allowed to dry overnight. On the following morning, 0.2 ml. of a 0.5%, 1%, 2% or 4% solution of glutaric acid in ethyl alcohol was applied to the circled area of the skin and distributed uniformly over the area with a sterile glass spatula. After a time interval (30 min., 1 hr., 2 hr., 4 hr. or 6 hr.) 0.1 ml. of undiluted virus was added to each test area and spread evenly over the area with a sterile glass spatula. The virus was allowed to remain on the treated skin for ten minutes. Virus was recovered from the skin by firmly holding a sterile glass cylinder 3 cm. in diameter over the area and adding 5 ml. of maintenance medium (M-199) to the interior of the cylinder. The skin was rinsed by drawing the fluid into a pipette and expelling it vigorously over the area five times. The fluid was transferred to a sterile tube for viral assay. As a control for non-specific inactivation and efficiency of virus recovery, non-treated skin was similarly inoculated with virus and the virus was recovered after contact of ten minutes. To determine if the virus sampling and rinsing procedure resulted in a carryover of the virucidal test material into the assay system, rabbit skin was treated with an ethyl alcoholic glutaric acid solution but not inoculated with virus and after 30 minutes rinsed as described above. The rinse fluids were assayed in tissue culture system and microscopically examined for cytotoxicity. The following results were obtained.

| Glutaric Acid Concentrations % | % Reduction of Virus Titer | | | | |
|---|---|---|---|---|---|
| | 30 min. | 1 hr. | 2 hr. | 4 hr. | 6 hr. |
| 0.5 | 0 | 0 | 0 | 0 | 0 |
| 1 | >99.9 | 99.99 | 99.9 | 99.9 | |
| 2 | >99.9 | 99.99 | 99.99 | 99.99 | 99.99 |
| 4 | | 99.99 | 99.99 | 99.99 | 99.99 |

Hard Surface Test for Residual Virucidal Activity Against Rhinovirus Type 2

Three-inch by five-inch Formica plates were cleansed by washing with Ivory soap and water, rinsing thoroughly under running tap water, then rinsing with 70% ethyl alcohol for ten minutes, finally rinsing with sterile distilled water and draining and drying with Formica side down overnight. Two-tenths ml. of a 0.5%, 1% or 2% ethyl alcoholic solution of glutaric acid was evenly distributed over a circular area 5 cm. in diameter and left to dry. After a time (30 min., 1 hr., 2 hr., 4 hr. or 6 hr.) 0.1 ml. of undiluted virus was applied to the test area and spread evenly over the surface with a sterile glass spatula. The virus was allowed to remain on the treated surface for ten minutes, then washed into a sterile petri dish with 5 ml. of maintenance medium (M-199). The wash fluid was transferred from the petri dish to a sterile tube with a sterile pipette and back to the petri dish and forth to the sterile tube four times, then titrated for viral content and/or cytotoxicity. The following results were obtained.

|  | % Reduction of Virus Titer | | | | |
| Glutaric Acid Concentrations % | 30 min. | 1 hr. | 2 hr. | 4 hr. | 6 hr. |
| --- | --- | --- | --- | --- | --- |
| 0.5 | >99.99 | >99.99 | >99.99 | >99.99 | |
| 1 | 99.99 | 99.99 | 99.999 | 99.999 | 99.999 |
| 2 | 99.99 | 99.99 | 99.999 | 99.999 | 99.999 |

## Hard Surface Test for Virucidal Activity Against Other Rhinoviruses

Four-inch by six-inch Formica plates were washed and rinsed in sterile distilled water and air-dried. The plates were contaminated by spreading 0.1 ml. of undiluted virus over a two-inch square area with a sterile microscope slide and left to dry. Ten minutes later 0.2 ml. of a 0.5%, 1% or 2% solution of glutaric acid in maintenance medium (M-199) was applied evenly to the contaminated area. The virus and glutaric acid were left in contact for ten minutes, then washed into a sterile petri dish with 5 ml. of maintenance medium (M-199). The wash fluid was transferred from the petri dish to a sterile tube with a sterile pipette and the washing and transferring steps were repeated four more times. The combined washes were titrated for viral content and/or cytotoxicity. Titers of all seven viruses were reduced by >99.99% by the 2% and 1% concentrations of glutaric acid. Titers of the seven viruses were reduced by the 0.5% concentration of glutaric acid as follows:

| Rhinovirus Type | % of Reduction of Virus Titer |
| --- | --- |
| 5 | >99.0 |
| 13 | >99.9 |
| 14 | >99.9 |
| 22 | >99.9 |
| 39 | 90 |
| 41 | >99.99 |
| 50 | >99.99 |

## Test of Glutaric Acid for Virucidal Activity Against Influenza Viruses

In these tests influenza virus A2/Japan/170/62 (6th egg passage in allantoic fluid, $EID_{50}$ $\log_{10}$ 7.5), influenza virus A0/PR8/34 (6th egg passage in allantoic fluid, $EID_{50}$ $\log_{10} \geq 9.0$), ten-day old embryonated chicken (White Leghorn) eggs and a commercial grade of glutaric acid were used.

Five-tenths ml. of undiluted virus was mixed with 4.5 ml. of a 1%, 2% or 4% solution of glutaric acid and sterile distilled water. The mixture was allowed to stand at ambient temperature for ten minutes and was then rapidly diluted by ten-fold steps in phosphate-buffered saline to a final dilution of $10^{-7.0}$. To test cytotoxicity the 1%, 2% and 4% solutions of glutaric acid were diluted 9:1 with sterile phosphate-buffered saline, allowed to stand 10 minutes at ambient temperature, and diluted by ten-fold steps in phosphate-buffered saline to a final dilution of $10^{-7.0}$.

Two-tenths ml. of each dilution was inoculated into the allantoic sac of each of five eggs. The eggs were incubated at 36.5°C. for 40 hours, then chilled overnight at 4°C. One-half-ml. of allantoic fluid was removed from each egg and mixed with 0.5 ml. of phosphate-buffered saline in tubes. One ml. of 0.75% washed chicken red blood cells was added to each tube. The tubes were shaken and allowed to stand at ambient temperature for 40 minutes. Hemagglutination patterns were read and the 50% egg infectious dose ($EID_{50}$) was calculated for each concentration of glutaric acid by the method of Reed and Muench (American Journal of Hygiene, vol. 27, pp. 493—497, 1938). Cytotoxicity of glutaric acid was evaluated by examining eggs for effect on the chick embryo viability.

All three concentrations of glutaric acid reduced virus titers by >99.99%. The titers of both viruses were reduced to non-detectable levels. None of the three concentrations of glutaric acid was found to be cytotoxic.

## Test of Glutaric Acid for Virucidal Activity Against Other Viruses

In tests similar to the hard surface test against rhinoviruses types 5, 13, 14, 22, 39, 41 and 50 described above, glutaric acid was tested against eight other viruses, as follows.

| Virus | Source | $Log_{10}$ per $TCID_{50}$ 0.2 ml. of Virus |
|---|---|---|
| Parainfluenza Type 3 (Soret) HA-1 Strain C-24 | Upjohn | 7.3 |
| Respiratory Syncytial Log Strain | ATCC | 5.2 |
| Poliovirus Type III Leon (Led.) MK10$^+$ | New York State Dept of Health | 7.5 |
| Poliovirus Type II YSK (Led.) Mk7$^+$ LLCL | New York State Dept. of Health | 7.5 |
| ECHO 9 (No. 5591) | New York State Dept. of Health | 7.0 |
| ECHO 11 (No. 5593) | New York State Dept. of Health | 7.0 |
| Herpes Simplex Type 1 (Sheely) | Francis J. Proctor Foundation, Univ. of California | 6.5 |
| Herpes Simplex Type 2 (Curtis) | Francis J. Proctor Foundation, Univ. of California | 6.5 |

The parainfluenza virus and poliovirus were maintained in a continuous line of HeLa (Ohio) cells. The respiratory syncytial virus, ECHO viruses and herpes simplex viruses were maintained in a continuous line of BSC-1 (monkey Kidney) cells.

The results of the tests showed that the minimum concentration of glutaric acid necessary to reduce the titers of the herpes simplex viruses types 1 and 2 by >99.99% was 1%, that the minimum concentration of glutaric acid necessary to reduce the titers of respiratory syncytial virus by >99.99% was 2%, and the minimum concentration of glutaric acid necessary to reduce the titer of parainfluenza virus type 3 by >99.99% was 4%. The results also showed that a 4% concentration of glutaric acid was not virucidal against polioviruses types II and III and ECHO viruses types 9 and 11.

The invention is intended for topical virucidal use both *in vitro* (on a non-living surface) and *in vivo*, (on human or other animal tissue) especially for use on the hands and especially for preventing transmission of rhinoviruses. For these purposes the glutaric acid can be formulated in any appropriate vehicle, provided that the glutaric acid and the vehicle are compatible, that is, that the virucidal activity of the glutaric acid is not diminished by the vehicle. Thus, the compositions can be in the form of creams, foams, lotions, ointments, solutions or sprays. The vehicles can be aqueous or non-aqueous, for example alcoholic or oleaginous, or a mixture thereof, and may additionally contain surfactants, emollients, lubricants, stabilizers, dyes, perfumes and preservatives. Conventional methods are used in preparing the compositions.

The foregoing compositions can be dispensed in premoistened pads or tissues. The latter can be packaged individually as described, for example, in U.S. Pat. 3,057,467 or multiply, in separate sheets as described, for example, in U.S. Pat. 3,836,044 or in a roll as described, for example, in U.S. Pat. 4,017,002.

## Example 1

The following example is a composition intended for use as a virucidal hand lotion wherein the vehicle is an aqueous surfactant-emollient mixture.

| Ingredient | Percent by Weight |
|---|---|
| Glutaric Acid | 1.00xxx |
| Glyceryl Stearate (and) PEG-100 Stearate | 3.00xxx |
| PPG-12-Buteth-16 | 2.00xxx |
| Cetyl Alcohol | 1.50xxx |
| Myristyl Alcohol | 1.50xxx |
| PEG-4 Laurate | 1.00xxx |
| PEG-4 Stearate | 0.500xx |
| Glycerin | 0.500xx |
| Perfume | 0.300xx |
| Carbomer-934P | 0.200xx |
| Zinc Pyrithione | 0.100xx |
| Dye | 0.00200 |
| Sodium Hydroxide to adjust pH to 5.5 | |
| Water to make | 100.00xxx |

Example 2

The composition corresponding to the composition of Example 1 except that 2.00% glutaric acid was substituted for 1.00% glutaric acid was also prepared.

Example 3

The following example is a composition intended for use as a virucidal foam wherein the vehicle is an aqueous ethyl alcoholic surfactant-emollient aerosol mixture.

| Ingredient | Percent by Weight |
|---|---|
| Glutaric Acid | 1.00xx |
| Steareth-2 | 1.50xx |
| Laneth-16 | 1.00xx |
| Cetyl Alcohol | 0.800x |
| Myristyl Alcohol | 0.200x |
| Perfume | 0.0500 |
| Alcohol, USP | 54.2xxx |
| Purified Water | 36.3xxx |
| Propane | 0.800x |
| Isobutane | 4.20xx |
| Water to make | 100.0xxx |

Example 4

A composition corresponding to the composition of Example 3 except that 2.00% glutaric acid was substituted for 1.00% glutaric acid was also prepared.

6

**0 008 121**

Tests of the Compositions Against Rhinovirus Type 2
*Rabbit Skin Test for Residual Virucidal Activity*

The compositions of the foregoing examples were tested in the rabbit skin test described above except that 0.4 ml. of the compositions of Examples 1 and 2 and a three-quarter inch ball of foam of the compositions of Example 3 and 4 were used and the compositions and virus were spread on the test site with a gloved finger instead of a sterile glass spatula. The following results were obtained.

% Reduction of Virus Titer

| Example | 30 min. | 1 hr. | 2 hr. | 4 hr. | 6 hr. |
|---|---|---|---|---|---|
| 1 | 99.9 | 99.9 | 99.9 | 99.9 | |
| | 99.9 | 99.9 | 99.9 | 99.9 | 99.9 |
| 2 | >99.9 | >99.9 | >99.9 | >99.9 | |
| | >99.9 | >99.9 | >99.9 | >99.9 | >99.9 |
| 3 | >99.9 | >99.9 | >99.9 | >99.9 | >99.9 |
| | >99.9 | >99.9 | >99.9 | >99.9 | >99.9 |
| 4 | >99.9 | >99.9 | >99.9 | >99.9 | >99.9 |

*Monkey Paw Test for Residual Virucidal Activity*

The paws of rhesus monkeys sedated with phencyclidene (Sernylan) were washed for 1 minute with Ivory soap and a brush, rinsed under running tap water for 30 seconds, dried with paper towels, rinsed with 70% ethyl alcohol, and allowed to dry for 15 minutes. Four-tenths ml. of the composition of Example 1 or 2 or a three-quarter inch ball of foam of the composition of Example 3 or 4 was placed on each paw and spread with a gloved finger. After a time interval (30 min., 1 hr., 2 hr., or 4 hr.) 0.1 ml. of undiluted virus was placed on each paw and spread over the palm with a glass spatula. After 10 minutes residual virus was recovered by placing the paw into a three-inch by eight-inch polyethylene bag containing 10 ml. of maintenance medium (M-199). The bag was squeezed repeatedly for 10 seconds to insure removal of virus from the paw, and the rinse fluid was transferred to sterile tubes pending assay.

To determine if the virus sampling and rinsing procedure resulted in a carry-over of the glutaric acid into the assay system, monkey paws were treated with each preparation, but not inoculated with virus, and after 30 minutes rinsed as described above. The rinse fluids were added to tissue culture and microscopically examined for cytotoxicity. Preliminary studies also showed that the amounts of glutaric acid in the recovery fluids were insufficient to inactivate rhinovirus type 2. The following results were obtained.

% Reduction of Virus Titer

| Example | 30 min. | 1 hr. | 2 hr. |
|---|---|---|---|
| 1 | >99.0 | >99.0 | >99.0 |
| 2 | >99.0 | >99.0 | >99.9 |
| 3 | 90 | 90 | 90 |
| 4 | >99.9 | >99.9 | >99.9 |

**Claims**

1. A process of neutralizing or destroying a susceptible virus present on a non-living surface or on a living human tissue or non-human animal tissue, said tissue having the virus located thereon but not infected by the virus for the purpose of preventing transmission of the virus, which comprises contacting the virus with a virucidally effective amount of glutaric acid.

2. A process according to claim 1, wherein the virus is rhinovirus, a herpes simplex virus, an influenza virus, a parainfluenza virus or a respiratory syncytial virus.

3. A process according to claim 1 or 2, in which the glutaric acid is contained in a vehicle.

7

4. A process according to claim 3 wherein the vehicle is an aqueous surfactant-emollient mixture.

5. A process according to claim 4, wherein the vehicle is an aqueous ethyl alcoholic surfactant-emollient aerosol mixture.

6. A composition for providing a virucidal effect against a susceptible virus, said composition being suitable for application to a human tissue or a non-human animal tissue, said tissue having the virus located thereon but not infected by the virus for the purpose of preventing transmission of the virus, said composition consisting essentially of a virucidally effective concentration of glutaric acid and an aqueous surfactant-emollient mixture.

7. A composition according to claim 6, wherein the vehicle is an aerosol mixture.

8. A composition according to claim 6, which is contained in pre-moistened tissues.

**Revendications**

1. Procédé de neutralisation ou de destruction d'un virus sensible présent sur une surface inanimée ou sur un tissu humain ou un tissu animal non humain vivant, le virus étant situé sur le tissu mais le tissu n'étant pas infecté par le virus, dans le but d'empêcher la transmission du virus, qui consiste à mettre le virus en contact d'une quantité efficace sur le plan virucide d'acide glutarique.

2. Procédé selon la revendication 1, où le virus est un rhinovirus, un virus herpes simplex, un virus grippal, un virus paragrippal ou un virus respiratoire syncytial.

3. Procédé selon la revendication 1 ou 2, où l'acide glutarique est contenu dans un véhicule.

4. Procédé selon la revendication 3, où le véhicule est un mélange aqueux émollient-surfactif.

5. Procédé selon la revendication 4, où le véhicule est un mélange aérosol aqueux-alcool éthylique surfactif-émollient.

6. Composition destinée à fournir un effet virucide sur un virus sensible, ladite composition convenant à l'application à un tissu humain ou un tissu animal non humain, le virus étant localisé sur ledit tissu mais le tissu n'étant pas infecté par le virus, dans le but d'empêcher la transmission du virus, ladite composition comprenant essentiellement un concentration efficace sur le plan virucide d'acide glutarique et un mélange aqueux surfactif-émollient.

7. Composition selon la revendication 6, où le véhicule est un mélange aérosol.

8. Composition selon la revendication 6, contenue dans des serviettes pré-humidifiées.

**Patentansprüche**

1. Verfahren zum Neutralisieren oder Zerstören eines empfindlichen Virus, der auf einer unlebendigen Fläche oder auf einem lebenden menschlichen oder nichtmenschlichen tierischen Gewebe vorhanden ist, sofern der Virus das Gewebe, auf dem er vorhanden ist, nicht infiziert hat, wobei eine Übertragung des Virus verhindert werden soll, dadurch gekennzeichnet, dass der Virus mit einer viruzidal wirksamen Menge Glutarsäure in Berührung gebracht wird.

2. Verfahren nach Anspruch 1, dadurch gekennzeichnet, dass der Virus ein Schnupfenvirus, ein Herpes simplex-Virus, ein Grippevirus, ein Parainfluenzavirus oder ein die Atmungsorgane befallender Synzitialvirus ist.

3. Verfahren nach Anspruch 1 oder 2, dadurch gekennzeichnet, dass die Glutarsäure in einem Trägermedium enthalten ist.

4. Verfahren nach Anspruch 3, dadurch gekennzeichnet, dass das Trägermedium ein wäßriges Tensid-Weichmacher-Gemisch ist.

5. Verfahren nach Anspruch 4, dadurch gekennzeichnet, dass das Trägermedium ein wäßrigäthylalkoholisches Tensid-Weichmacher-Gemisch ist.

6. Stoffzusammensetzung zum Ausüben einer virucidalen Wirkung gegen einen empfindlichen Virus, wobei die Stoffzusammensetzung für das Auftragen auf ein menschliches Gewebe oder ein Nichtmenschliches tierisches Gewebe geeignet ist, auf dem sich der Virus befindet, das aber von dem Virus nicht infiziert ist, und um eine Übertragung des Virus zu verhindern, dadurch gekennzeichnet, dass die Stoffzusammensetzung in wesentlichen aus Glutarsäure in einer viruzidal wirksamen Konzentration und einem wäßrigen Tensid-Weichmacher-Gemisch besteht.

7. Stoffzusammensetzung nach Anspruch 6, dadurch gekennzeichnet, dass das Trägermedium eine Aerosolgemisch ist.

8. Stoffzusammenzetzung nach Anspruch 6, dadurch gekennzeichnet, dass sie in vorangefeuchteten Geweben enthalten ist.